# EUROPEAN PATENT APPLICATION

(11) **EP 4 760 739 A1**
(43) Date of publication of application: **17.06.2026**
(21) Application number: 25203143.0
(22) Date of filing: 18.09.2025
(51) Int. Cl.: G16H 20/70, G16H 50/70

(54) **SYSTEMS AND METHODS FOR GENERATING HEALTH DISORDER SUMMARY AND RECOMMENDATIONS USING LARGE LANGUAGE MODELS**

(30) Priority: 10.12.2024 IN 202421097230
(71) Applicant: Tata Consultancy Services Limited, Mumbai, Maharashtra 400 021 (IN)
(72) Inventor: CHATTERJEE, Arnab, 110016 New Delhi (IN); SACHIN, Sachin, 110016 New Delhi (IN); AGGARWAL, Vaishali, 110016 New Delhi (IN)
(74) Representative: Goddar, Heinz J.

(57) **Abstract**

Textual data from social platforms captures various aspects of mental health through discussions around and across issues, while users reach out for help and others sympathize and offer support. Present disclosure leverages Natural Language Processing (NLP) and Generative AI techniques to identify and assess mental health disorders, detect their severity, and create recommendations for behavior change and therapeutic interventions based on input data associated with users. To classify the disorders, the system leverages Large Language Models (LLMs) to filter relevant data from input data, the relevant data pertains to health disorder of users. Further, from the relevant data, various features are extracted to create user profiles and different summaries. The summaries are then aggregated to obtain a final summary for generation of actionable health disorder specific recommendations.

## Description

### CROSS-REFERENCE TO RELATED APPLICATIONS AND PRIORITY

The present application claims priority from Indian patent application no. 202421097230, filed on December 10, 2024.

### TECHNICAL FIELD

The disclosure herein generally relates to text analysis, and, more particularly, to systems and methods for generating health disorder summary and recommendations using Large Language Models.

### BACKGROUND

Mental health is crucial for overall well-being, with early detection and intervention being vital. Mental illness affects millions worldwide and is a significant health burden, worsened by the COVID-19 pandemic. Social media offers valuable insights into mental health through extensive user discussions around experiences and struggles. However, analyzing this data is challenging due to informal language, slang, and emojis, which complicate traditional natural language processing (NLP) techniques. Despite these challenges, social media provides important insights and requires efficient, scalable analysis methods.

Generative AI (GenAI), especially Large Language Models (LLMs), has greatly improved the analysis of unstructured text data by understanding and generating human-like text. However, for mental health data, one needs to be careful about misinformation and misdiagnosis due to the potential for generating convincing but false content. Ethical issues, biases, and the need for human oversight are important considerations.

### SUMMARY

Embodiments of the present disclosure present technological improvements as solutions to one or more of the above-mentioned technical problems recognized by the inventors in conventional systems.

For example, in one aspect, there is provided a processor implemented method for generating health disorder summary and recommendations using Large Language Models. The method comprises receiving, via one or more hardware processors, an input data pertaining to a plurality of users; filtering, by using one or more Large Language Models (LLMs) via the one or more hardware processors, the input data to obtain a filtered data specific to at least one health disorder pertaining to at least a subset of the plurality of users; extracting, by using the one or more LLMs via the one or more hardware processors, a first set of features and a second set of features from the filtered data based on one or more context associated prompts; creating, by using the one or more LLMs via the one or more hardware processors, one or more user profiles for the subset of the plurality of users based on the extracted first set of features and the extracted second set of features; generating a first summary and a second summary based on the extracted first set of features and the extracted second set of features respectively for each user profile amongst the one or more user profiles; generating, by using the one or more LLMs via the one or more hardware processors, a third summary based on the first summary and the second summary; generating, by using the one or more LLMs via the one or more hardware processors, one or more actionable health disorder specific recommendations based on an analysis of the third summary.

In an embodiment, the first set of features comprises at least one of (i) a duration, (ii) a frequency, (iii) a recurrence, and (iv) one or more explicit time and date mentions.

In an embodiment, the second set of features comprises at least one of (i) a severity of the at least one health disorder, (ii) one or more factors impacting the at least one health disorder, and (iii) a language and tonality in the filtered data pertaining to the at least one health disorder.

In an embodiment, the one or more actionable health disorder specific recommendations comprise at least one of a therapy, and one or more behavioral changes.

In another aspect, there is provided a processor implemented system for generating health disorder summary and recommendations using Large Language Models. The system comprises: a memory storing instructions; one or more communication interfaces; and one or more hardware processors coupled to the memory via the one or more communication interfaces, wherein the one or more hardware processors are configured by the instructions to receive an input data pertaining to a plurality of users; filter, by using one or more Large Language Models (LLMs), the input data to obtain a filtered data specific to at least one health disorder pertaining to at least a subset of the plurality of users; extract, by using the one or more LLMs, a first set of features and a second set of features from the filtered data based on one or more context associated prompts; create, by using the one or more LLMs, one or more user profiles for the subset of the plurality of users based on the extracted first set of features and the extracted second set of features; generate, by using the one or more LLMs, a first summary and a second summary based on the extracted first set of features and the extracted second set of features respectively for each user profile amongst the one or more user profiles; generate, by using the one or more LLMs, a third summary based on the first summary and the second summary; generate, by using the one or more LLMs, one or more actionable health disorder specific recommendations based on an analysis of the third summary.

In an embodiment, the first set of features comprises at least one of (i) a duration, (ii) a frequency, (iii) a recurrence, and (iv) one or more explicit time and date mentions.

In an embodiment, the second set of features comprises at least one of (i) a severity of the at least one health disorder, (ii) one or more factors impacting the at least one health disorder, and (iii) a language and tonality in the filtered data pertaining to the at least one health disorder.

In an embodiment, the one or more actionable health disorder specific recommendations comprise at least one of a therapy, and one or more behavioral changes.

In yet another aspect, there are provided one or more non-transitory machine-readable information storage mediums comprising one or more instructions which when executed by one or more hardware processors cause generating health disorder summary and recommendations using Large Language Models by receiving an input data pertaining to a plurality of users; filtering, by using one or more Large Language Models (LLMs), the input data to obtain a filtered data specific to at least one health disorder pertaining to at least a subset of the plurality of users; extracting, by using the one or more LLMs, a first set of features and a second set of features from the filtered data based on one or more context associated prompts; creating, by using the one or more LLMs, one or more user profiles for the subset of the plurality of users based on the extracted first set of features and the extracted second set of features; generating, by using the one or more LLMs, a first summary and a second summary based on the extracted first set of features and the extracted second set of features respectively for each user profile amongst the one or more user profiles; generating, by using the one or more LLMs, a third summary based on the first summary and the second summary; generating, by using the one or more LLMs, one or more actionable health disorder specific recommendations based on an analysis of the third summary.

In an embodiment, the first set of features comprises at least one of (i) a duration, (ii) a frequency, (iii) a recurrence, and (iv) one or more explicit time and date mentions.

In an embodiment, the second set of features comprises at least one of (i) a severity of the at least one health disorder, (ii) one or more factors impacting the at least one health disorder, and (iii) a language and tonality in the filtered data pertaining to the at least one health disorder.

In an embodiment, the one or more actionable health disorder specific recommendations comprise at least one of a therapy, and one or more behavioral changes.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 depicts an exemplary system for generating health disorder summary and recommendations using Large Language Models, in accordance with an embodiment of the present disclosure.
FIG. 2 depicts an exemplary high level block diagram of the system of FIG. 1 for generating health disorder summary and recommendations using Large Language Models, in accordance with an embodiment of the present disclosure.
FIG. 3 depicts an exemplary flow chart illustrating a method for generating health disorder summary and recommendations using Large Language Models of the systems of FIG. 1-2, in accordance with an embodiment of the present disclosure.
FIG. 4 depicts a graphical representation illustrating used language styles and tones by online users, in accordance with an embodiment of the present disclosure.
FIG. 5 depicts a graphical representation illustrating distribution of various therapies recommended to the users, in accordance with an embodiment of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Analyzing social media data for mental health diagnosis has become increasingly significant due to the wealth of information available on social media platforms such as Twitter^{®}, Reddit^{®}, etc. The field has seen significant advancements, with various methodologies developed to enhance the accuracy and efficiency of identifying mental health conditions from user generated content. Linguistic markers of depression examined from Twitter data highlighted the potential of social media for mental health insights. Recent work on identifying stress factors that lead to mental health decline has demonstrated the effectiveness of NLP techniques in this domain. Various machine learning techniques have been used to detect mental health issues on social media platforms, where deep learning methods enhanced detection accuracy. "Dreaddit", a dataset for stress analysis in social media posts, was used to demonstrate the efficacy of NLP techniques for identifying stress-related content on Reddit using binary classification.

Another literature work discussed a gold standard dataset for detecting depression levels from social media posts using traditional algorithms and data augmentation in a multi-class classification framework. Bidirectional Encoder Representations from Transformers (BERT) has set new NLP benchmarks by employing bidirectional transformer training to better understand language context than earlier models. Yet another literature, RoBERTa (Robustly optimized BERT approach) improves upon BERT by optimizing the pre-training phase. Analyzing the literature, it is found that LLMs resemble low-quality therapy behaviors but also reflect more on clients' needs and strengths, indicating the need for further research to ensure high-quality care.

Embodiments of the present disclosure provide a system and a method that leverage the knowledge of Large Language Models (LLMs) to (i) identify mental health issues by utilizing social media data to detect whether it is related to mental health, (ii) classify disorders and severity, and (iii) recommend interventions for (i) behavior changes and (ii) therapies based on the identified disorders and their severity. More specifically, the system and method of the present disclosure address the lack of accessible, scalable, and cost-effective mental health support by leveraging LLMs to provide early detection, diagnosis using widely accepted international psychiatric standards, and personalized recommendations. Using mental health related user text as input, the system automatically generates different features along with recommendations to be used by mental health practitioners as well as organizations. It benefits practitioners by aiding in diagnosis and reducing workload, while helping corporations by enhancing employee wellbeing, providing personalized support, and offering data-driven insights, leading to a healthier and more proactive work environment, thereby positively impacting productivity.

This addresses gaps in automated, personalized mental health assessment by integrating both temporal and non-temporal information/features for assessment using international Psychiatric standards (e.g., DSM5), from text inputs. The system of the present disclosure provides context-aware, scalable recommendations and ensures privacy, improving the accuracy and relevance of mental health support while overcoming limitations in current/conventional solutions.

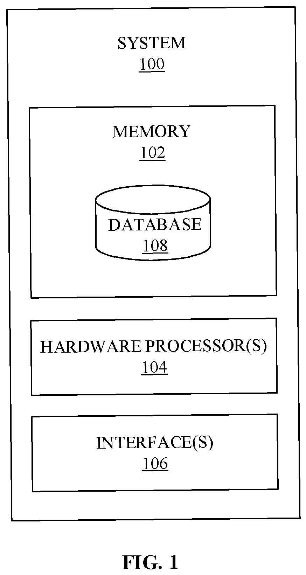

Referring now to the drawings, and more particularly to FIGS. 1 through 5, where similar reference characters denote corresponding features consistently throughout the figures, there are shown preferred embodiments, and these embodiments are described in the context of the following exemplary system and/or method.

FIG. 1 depicts an exemplary system 100 for generating health disorder summary and recommendations using Large Language Models, in accordance with an embodiment of the present disclosure. In an embodiment, the system 100 includes one or more hardware processors 104, communication interface device(s) or input/output (I/O) interface(s) 106 (also referred as interface(s)), and one or more data storage devices or memory 102 operatively coupled to the one or more hardware processors 104. The one or more processors 104 may be one or more software processing components and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is/are configured to fetch and execute computer-readable instructions stored in the memory. In an embodiment, the system 100 can be implemented in a variety of computing systems, such as laptop computers, notebooks, hand-held devices (e.g., smartphones, tablet phones, mobile communication devices, and the like), workstations, mainframe computers, servers, a network cloud, and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random-access memory (SRAM) and dynamic-random access memory (DRAM), and/or non-volatile memory, such as read only memory (ROM), erasable programmable ROM, flash memories, hard disks, optical disks, and magnetic tapes. In an embodiment, a database 108 is comprised in the memory 102, wherein the database 108 comprises input data received from one or more sources (e.g., social media, and the like). The input data pertains to health information of a plurality of users. The database 108 further comprises filtered data that is specific to health disorders of one or more users, and the like. The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

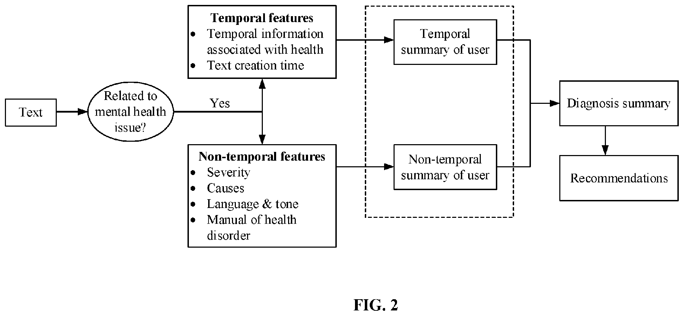

FIG. 2, with reference to FIG. 1, depicts an exemplary high level block diagram of the system 100 of FIG. 1 for generating health disorder summary and recommendations using Large Language Models, in accordance with an embodiment of the present disclosure.

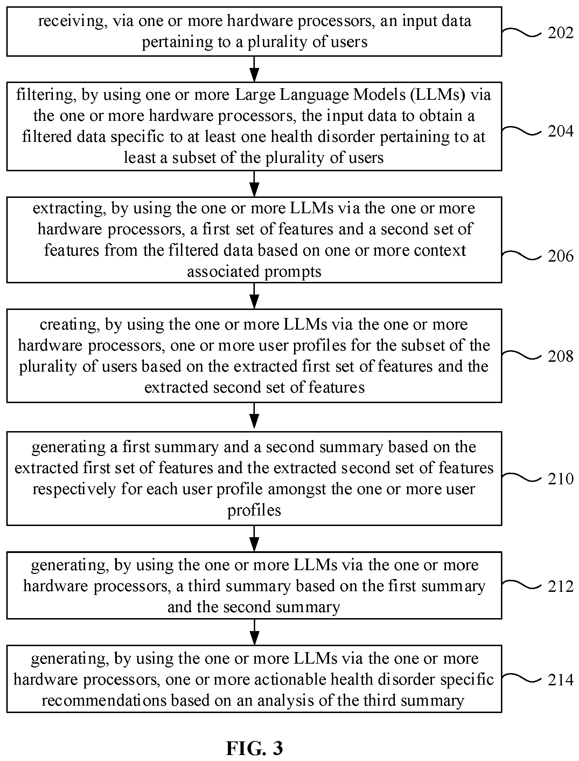

FIG. 3, with reference to FIGS. 1-2, depicts an exemplary flow chart illustrating a method for generating health disorder summary and recommendations using Large Language Models of the systems 100 of FIG. 1-2, in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104. The steps of the method of the present disclosure will now be explained with reference to components of the system 100 of FIG. 1, the block diagram of the system 100 depicted in FIG. 2, and the flow diagram as depicted in FIG. 3. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

At step 202 of the method of the present disclosure, the one or more hardware processors 104 receive an input data pertaining to a plurality of users. The input data may comprise at least one of one or more posts, one or more comments from the plurality of users. This input data may be obtained through various one or more sources such as websites, community forums, social media, and the like. Below Table 1 depicts exemplary input data received from the one or more sources:

**Table 1**

| Text | Author/User | Time/date |
|---|---|---|
| It can which is why I have two accounts and only on this one I follow the depression thread . The ones that make most depressed is when people post about how their life is getting amazing and it gets | jay-sid- | 2020-04-03 05:45:20 |
| A bunch of upvotes . Like that's great you're getting better but I haven't been able to in 5 years and just don't want to see that | | |
| well, if you do tell a teacher or friend, tell them how you feel about others as well. why you don't like reaching out. maybe that'd help them understand your feelings more. but it's entirely up to you. i wish you the best regardless. | testamentsofanguish | 2019-12-02 11:57:58 |
| great job! | testamentsofanguish | 2019-12-15 23:46:34 |
| i am so lonely and you would think with the internet there wouldn't be anymore loneliness. but yet here i am 39, no friends, no family, playing world of warcraft and absolutely no one to spend time with or talk too and absolutely alone even in a mmo. didn't think it was possible but | d3ad1ns1de | 2020-05-02 01:37:52 |
| surely it is. the loneliness is killing me, seeing all these couples having someone with them and doing things with and sharing things with and yet here i sit can't even get a simple date or even a woman who is interested in talking to me that doesn't ghost me after an hour or two or a day or two. i am so tired of the loneliness. its destroying me and playing with my mind badly. | | |
| was feeling this the past few days, made some posts, deleted them, did some other things and realise what i'm feeling isn't normal and i'm not faking it to myself | Smarmy Slayer | 2020-02-20 06:18:46 |
| is there a way to die without causing family and friends plain? | SubstanceFederal | 2020-07-29 15:11:50 |
| you don't need to feel lonely. we are there to | kindofbrill | 2020-06-23 16:49:52 |
| make you feel the complete opposite. we are always here to support and help you, no matter what. whenever you feel comfortable, sending myself a dm and we'll talk. :) | | |
| that's amazing! ahhh i'm so happy for you! :) | ambition786 | 2020-03-07 16:50:10 |
| sleep issues? idk i woke up feeling upset and sick. just mad at myself. honestly this happens every single time i nap and has been a thing since i was a kid. short periods of sleep tick me off. is this odd? feels odd. | ambition786 | 2020-07-19 09:47:25 |
| it's hard to work on things i like or want to do i don't think i've ever stuck to any long term project that i wanted to do or liked for myself. most of the time, i've only ever done things that i had to do in life. i am not sure i | carter_pride | 2020-06-01 2:44:44 |
| could ever do anything for myself and then stay committed to it. that's the life of a slave, a soldier, and an oppressed immigrant. and i am not sure i can snap out of it. maybe it's time to see a therapist after all. | | |

At step 204 of the method of the present disclosure, the one or more hardware processors 104 filter, by using the one or more LLMs, the input data to obtain a filtered data specific to at least one health disorder pertaining to at least a subset of the plurality of users. The system 100 employs LLMs (Large Language Model) to filter out irrelevant content, thereby refining the input data to include only posts that are genuinely related to health conditions/mental health/health disorder. Each post is evaluated to determine whether it pertained to health issue, with the LLMs instructed to provide responses one or more pre-defined formats (e.g., say "yes" or "no" format). By leveraging the advanced capabilities of LLMs, the system 100 efficiently classifies/filters the vast number of posts, ensuring the integrity and focus of the analysis on meaningful health-related content. Below Table 2 is the filtering done to identify mental health related post using one or more designed prompt to obtain filtered data way of examples.

**Table 2**

| Text | System Prompt for Filtration | User Prompt for Filtration | Related to health disorder |
|---|---|---|---|
| It can which is why I have two | You are an advanced AI | | Yes |
| accounts and only on this one I follow the depression thread . The ones that make most depressed is when people post about how their life is getting amazing and it gets A bunch of upvotes . Like that's great you're getting better but I haven't been able to in 5 years and just don't want to see that | language model trained to classify and understand various human problems. Your task is to determine whether a described problem is related to a mental disorder or not. Use your knowledge of psychology, mental health, and diagnostic criteria such as those found in the DSM-5 (Diagnostic and Statistical Manual of Mental Disorders) to make your determination. Be sensitive and respectful in your analysis, | Problem: Text. Based on this information, do you think this problem is related to a mental disorder? Only give 'YES' or 'NO' answers nothing else. | |
| i feel so disconnected with reality I don't know how to explain it but I almost feel like everything is a dream at this point or just not real ? | | | Yes |
| Memories from the past feel like they happened in a different lifetime , really strange feeling | recognizing the complexity and nuances of mental health issues. | | |
| exactly ! thanks man , i hope the same for you | | | No |
| tonight's going to be rough | | | No |
| I know .. and it's so scary .. you Really feel like you're the only human being in this world | | | Yes |
| the realization that life can be taken from you at any moment is the reason i'm unable to enjoy anything | | | Yes |
| tonight's going to be rough | | | No |
| it helps me but also can make | | | No |
| things worse for me . | | | |

At step 206 of the method of the present disclosure, the one or more hardware processors 104 extract, by using the one or more LLMs, a first set of features and a second set of features from the filtered data based on one or more context associated prompts. The first set of features and the second set of features are different from each other, in one embodiment of the present disclosure. For instance, the first set of features comprises temporal features such (i) a duration, (ii) a frequency, (iii) a recurrence, and (iv) one or more explicit time and date mentions, or combinations thereof. The second set of features comprises (i) a severity of the at least one health disorder, (ii) one or more factors impacting the at least one health disorder, (iii) a language and tonality in the filtered data pertaining to the at least one health disorder, (iv) illness as mentioned in a health disorder manual (e.g., Diagnostic and Statistical Manual of Mental Disorders, and the like). The temporal and non-temporal features are extracted using one or more designed prompts which are provided below in Tables 3 through Table 6 along with the examples.
1. Temporal Features: Timeline
2. Non-Temporal Features: DSM-5 Disorder Classification, Severity, Language Style and Tone, Causes, and the like.

**Table 3**

| Text | System Prompt for DSM-5 Disorder Classification and Timeline | User Prompt for DSM-5 Disorder Classification and Timeline | DSM-5 Disorder Classification | Timeline |
|---|---|---|---|---|
| cried in the car today infront of my mom I canÔÇÖt even control my emotions anymore . In the past I could but now itÔÇÖs like I donÔÇÖt even care I just cry at work , around family . The advice people give me is just Itll get better and that everyone is sad . As if that makes me feel better . It sucks when the people closest to you truly donÔÇÖt understand what itÔÇÖs like living with this | You are an expert in clinical psychology, specialized in diagnosing mental health conditions based on textual analysis. Your task is to predict the potential mental health problems(one or more) the writer of the provided text is most likely and prominently suffering from, following the DSM-5(Diagnostic and Statistical Manual of Mental Disorders, Fifth Edition) criteria. Provide only the names of the problems that are MOST RELEVANT AND STRONGLY SUPPORTED by the content in the text, keeping your conclusions within the framework of DSM-5 criteria. In case, some of the problems can be combined into one major class, return the name of the major class in the output. Do not give any explanations or reasons. Additionally, you must identify any mention of time periods that indicate how often the condition occurs, how long it has been prevailing, or any other relevant temporal information. If no clear time frame is present, give 'No Timeline' as an output. Do not give any explanations or reasons. Output Format: - If No timeline is mentioned: Potential Mental health conditions - names of the mental health problems predicted separated by commas; Timeline - No Timeline - If timeline is mentioned: Potential Mental health conditions - names of the mental health problems predicted separated by commas; Timeline - timeline period mentioned. | Analyze the following text to predict the potential mental health conditions the writer might be suffering from, based on DSM-5 criteria. Also, identify any references to time periods that indicate the frequency or duration of the symptoms. Do not provide any explanations or reasons. Provide the output in the specified output format only. Writer's text: Text | Major Depressive Disorder, Borderline Personality Disorder | No Timeline |
| Meds? Does anyone know a good over the counter med to help calm down? I having the roughest time in years just trying to keep myself grounded and it's been getting worse over the last few weeks. | | | Generalized Anxiety Disorder, Adjustment Disorder | Last few weeks |

**Table 4**

| **Text** | **System Prompt for Severity Classification** | **User Prompt for Severity Classification** | **Severity** |
|---|---|---|---|
| cried in the car today infront of my mom I canÔÇÖt even control my emotions anymore . In the past I could but now itÔÇÖs like I donÔÇÖt even care I just cry at work , around family . The advice people give me is just Itll get better and that everyone is sad . As if that makes me feel better . It sucks when the people closest to you truly donÔÇÖt understand what itÔÇÖs like living with this | You are a highly experienced psychiatrist. Your task is to examine the given user mental health problem's severity considering DSM-5(Diagnostic and Statistical Manual of Mental Disorders, Fifth Edition) and classify it into one of three labels: 'mild', 'moderate', or 'severe'. "Respond strictly with one of these labels without providing any additional details. | Here is the description of a user's mental health problem: Text. Choose one from 'mild', 'moderate', or 'severe'. | Moderate |
| Meds? Does anyone know a good over the counter med to help calm down? I having the roughest time in years just trying to keep myself grounded and it's been getting worse over the last few weeks. | Only respond with the labels 'mild', 'moderate', or 'severe'. | | Moderate |

**Table 5**

| **\Text** | **System Prompt for Language Style and Tone** | **User Prompt for Language Style and Tone** | **Severity** |
|---|---|---|---|
| cried in the car today infront of my mom I canÔÇÖt even control my emotions anymore . In the past I could but now itÔÇÖs like I donÔÇÖt even care I just cry at work , around family . The advice people give me is just Itll get better and that everyone is sad . As if that makes me feel better . It sucks when the people closest to you truly donÔÇÖt understand what itÔÇÖs like living with this | You are an expert language model designed to analyze the language style and tone of the text provided. Your task is to analyze the given text to determine the major language style and tone used throughout. Focus on identifying patterns of language that indicate the writer's emotional and mental state. Provide the accurate or the most related words for those in the format - The overall language style and tone used is: [answer] | Text: Text.\textbackslash{}n Analyze the following text and identify the overall language style and tone present throughout and then summarize it in one line with accurate or the most related words.\n Do not Provide any other explanations or reasons. Just give on point answer. | Despondent and Frustrated |
| Meds? Does anyone know a good over the counter med to help calm down? I having the roughest time in years just trying to keep myself grounded and it's been getting worse over the last few weeks. | | | Informal and Anxious |

**Table 6**

| **Text** | **System Prompt for Causes** | **User Prompt for Causes** | **Severity** |
|---|---|---|---|
| cried in the car today infront of my mom I canÔÇÖt even control my emotions anymore . In the past I could but now itÔÇÖs like I donÔÇÖt even care I just cry at work , around family . The advice people give me is just Itll get better and that everyone is sad . As if that makes me feel better . It sucks when the people closest to you truly donÔÇÖt understand what itÔÇÖs like living with this | You are an advanced language model with expertise in mental health diagnosis and DSM-5 criteria. Your task is to extract and summarize the major factors that triggers the mental health disorder described in the text. Consider symptoms, context, and any references to factors triggering mentioned in the text.\n Do not provide any reasons or explanations. Directly provide the factors apart from the disorder triggering the mental health disorder separated by commas without any other reason or explanations in the format - The possible factors are: [answer] | Text: Text'. \n Please analyze the text and identify the major/potential factors that might trigger the mental health disorder described. Base your analysis on DSM-5 criteria and provide a concise summary of these factors apart from the disorders.\n Do not provide any reasons or explanations. Directly provide the factors triggering separated by commas without any other reason or explanations. | emotional dysregulation, lack of emotional control, stress, social isolation, lack of understanding and support from others, and feelings of being misunderstood. |
| Meds? Does anyone know a good over the counter med to help calm down? I having the roughest time in years just trying to keep myself grounded and it's been getting worse over the last few weeks. | | | stress, anxiety, lack of grounding, worsening over time, recent weeks. |

At step 208 of the method of the present disclosure, the one or more hardware processors 104 create, by using the one or more LLMs, one or more user profiles for the subset of the plurality of users based on the extracted first set of features and the extracted second set of features. The user profile creation for each user was done using python codes, in one example embodiment of the present disclosure. Below Table 7 depicts the user profile creation by the system 100 by way of examples:

**Table 7**

| **Tex t** | **Author** | **datetim e** | **DSM-5 Disorde r** | **Severity** | **Timelin e** | **Languag e Style and Tone** | **Causes** |
|---|---|---|---|---|---|---|---|
| Text 1 | Author 1 | Datetim e 1 | Disorder s classifie d using the above prompts | Severity level classifie d using the above prompts | Timelin e 1/ No Timelin e | Langauge Style and tone identified | Causes identifie d |
| Text 2 | Author 1 | Datetim e 2 | Disorder s classifie d using the above prompts | Severity level classifie d using the above prompts | Timelin e 2/ No Timelin e | Langauge Style and tone identified | Causes identifie d |
| Text 3 | Author 1 | Datetim e 3 | Disorder s classifie d using the above prompts | Severity level classifie d using the above prompts | Timelin e 3/ No Timelin e | Langauge Style and tone identified | Causes identifie d |
| • | • | • | • | • | • | • | • |
| • | • | • | • | • | • | • | • |
| • | • | • | • | • | • | • | • |
| Text n | Author 1 | Datetim e n | Disorder s classifie d using the above prompts | Severity level classifie d using the above prompts | Timelin e n/ No Timelin e | Langauge Style and tone identified | Causes identifie d |

At step 210 of the method of the present disclosure, the one or more hardware processors 104 generate, by using the one or more LLMs, a first summary (e.g., temporal summary of the user and may be interchangeably used herein) and a second summary (e.g., non-temporal summary of the user and may be interchangeably used herein based on the extracted first set of features and the extracted second set of features respectively for each user profile amongst the one or more user profiles. Below Table 8 through Table 11 depict examples of the first summary and the second summary generated by the system 100.

**Table 8**

| Non-Temporal Summary: The input text to generate a non-temporal summary for Author1 is shown below: | | | | | | |
|---|---|---|---|---|---|---|
| **Text** | **Author** | **Tag** | **DSM-5 Disorder** | **Severity** | **Language Style and Tone** | **Causes** |
| Text 1 | Author1 | post/comment | Disorders classified using the above prompts | Severity level classified using the above prompts | Langauge Style and tone identified | Causes identified |
| Text 2 | Author1 | post/comment | Disorders classified using the above prompts | Severity level classified using the above prompts | Langauge Style and tone identified | Causes identified |
| Text 3 | Author1 | post/comment | Disorders classified using the above prompts | Severity level classified using the above prompts | Langauge Style and tone identified | Causes identified |
| • | • | • | • | • | • | • |
| • | • | • | • | • | • | • |
| • | • | • | • | • | • | • |
| Text n | Author1 | post/comment | Disorders classified using the above prompts | Severity level classified using the above prompts | Langauge Style and tone identified | Causes identified |

**Table 9**

| System Prompt to generate Non-Temporal Summary | User Prompt to generate Non-Temporal Summary | Output (Non-Temporal Summary generated) |
|---|---|---|
| Analyze the dataset provided, which includes the following features: full_text (content), Tag (post or comment), Datetime (reporting date and time), Symptoms (potential mental health disorders), Severity (severity level based on content and disorders), and Timeline (temporal information). | Dataset : Dataframe.\n Given the dataset with the following columns: full_text, Tag, Datetime, Disorders, Severity, Language Style and Tone, and Causes, analyze the information to produce a summary of the user's mental health condition.\n 1. Identify key mental health disorders mentioned in the | { "Overall Severity": "Severe", "Major Causes": "Childhood trauma, family dynamics, repeated rejection, poverty, homelessness, lack of education, and social isolation", |
| | | "Overall Language Style and Tone": "Despondent, despairing, and hopeless, with a focus on self-referential content", |
| Based on these features and the DSM-5 criteria, generate a comprehensive summary of the user's mental health condition. \n The Output should include the following headers : 1. Severity \n 2. Causes \n 3. Language Style and Tone 4. Recurring Themes \n 5. Mental Health Disorders(DSM-5 Criteria) \n 6. Mental Health Status \n\n Ensure that the summary is concise, comprehensive, presented only in json format and limited to a maximum of 200 words. The summary should not consist any additional Note and any other information in the beginning or at the end. | Disorders column and compare them against DSM-5 criteria.\n 2. Assess the severity levels provided and related them to the potential impact on the user's overall mental health.\n 3. Evaluate the temporal data to highlight trends, including any worsening or improvement in the user's condition over time, analyzing trends over time.\n 4. Detect any pattern or significant behavioral changes indicated by the Severity and Datetime columns.\n 5. Identify the causes for mental health disorder inferred from the user's content.\n 6. Analysis of language style, tone, and significant patterns.\n The summary should be structured, cohesive, and suitable for further clinical evaluation or therapeutic recommendations. | "Recurring Themes": "Themes of rejection, isolation, and hopelessness are prevalent throughout the content", |
| | | "DSM-5 Criteria": "The author's symptoms align with DSM-5 criteria for Major Depressive Disorder, PTSD, and Adjustment Disorder", "Mental Health Status": "The author's mental health condition appears to be chronic and worsening, with a high risk of relapse" } |

### Input Text for Temporal Summary for Author1 :

All the entries do not contain Timeline information and some of them were marked as 'No Timeline' in 'Timeline' column of Author1 dataframe. For temporal summary, the rows for which the values were marked as 'No Timeline' were removed from the dataset and the remaining dataset was given as the input text. The input dataframe for Temporal Summary is as follows:

**Table 10**

| **Text** | **Author** | **datetime** | **Tag** | **Timeline** |
|---|---|---|---|---|
| Text 1 | Author1 | datetime1 | post/comment | Timeline 1 |
| Text 2 | Author1 | datetime2 | post/comment | Timeline 2 |
| Text 3 | Author1 | datetime3 | post/comment | Timeline 3 |
| • | • | • | • | • |
| • | • | • | • | • |
| • | • | • | • | • |
| Text k | Author1 | datetimek | post/comment | Timeline k |

**Table 11**

| System Prompt to generate Temporal Summary | User Prompt to generate Temporal Summary | Output (Temporal Summary generated) |
|---|---|---|
| You are an expert in mental health analysis with a focus on temporal data and its interpretation using DSM-5. Your task is to analyze the content posted by the user, specifically focusing on temporal information extracted from the 'Timeline' column and corresponding chronological patterns from the 'datetime' column. Ensure to summarize the timeline information in chronological order, both from the content of the post and the timeline of when the user posted it, and match it with relevant DSM-5 criteria covering | Dataset : Dataframe.\n Analyze the dataset provided, which contains three columns: 'full_text' (the content posted by the user), 'datetime' (the date and time when the content was posted), and 'Timeline' (temporal information extracted from the content).\n Summarize the temporal information from the 'Timeline' column and arrange it in chronological order based on both the 'datetime' and the content's temporal information. | "The user, beauteeedur, has posted a total of 5 entries, with the earliest post dated March 5, 2020, and the latest post dated May 14, 2020. The temporal information extracted from the 'Timeline' column and the 'full_text' content is summarized below in chronological order: |
| | | * At least 20 years (March 5, 2020): The user mentions struggling with depression for at least 20 years. |
| | | * Years on years on years on years (April 17, 2020): The user mentions experiencing depression for multiple years. |
| | | * Roughly year 20 (April 22, 2020): The user |
| every aspect in 500 words at the max. Output the summary in a structured format with: \n -Concise and Comprehensive summary of timeline of posts combining the in-text temporal features from the 'Timeline' column and the reporting date and time from the 'datetime' column and the corresponding context from the 'full_text' column, for instance, it carefully notes every temporal information giving us which incident happen when and when was it reported and what is the timeline information mentioned by the user in the text in the chronological order and | | confirms struggling with depression for approximately 20 years. |
| | | * Three years (May 14, 2020): The user mentions living for others and getting worried about themselves for the past three years. |
| | | * 15 years (May 14, 2020): The user mentions trying to escape reality and feeling "fucked" for 15 years." |
| | | "Temporal patterns" : "The temporal patterns observed from the 'Timeline' column and the 'full_text' content are: |
| | | * Duration: The user has been struggling with depression for at least 20 years, with some periods lasting for multiple years. |
| | | * Frequency: The user mentions experiencing |
| cross checking the timeline information with the content to know the reason of timeline mentioning.\n - Temporal patterns observed (duration, frequency, recurrence, specific mentions of time within the text taking from Timeline column and the date and time it was posted). Limit your output length to 500 words. | | depression for multiple years, with no specific mention of frequency. |
| | | * Recurrence: The user's symptoms seem to have recurred over the years, with no clear pattern of remission. |
| | | * Specific mentions of time: The user mentions specific time periods, such as 20 years, 15 years, and 3 years, indicating a sense of duration and chronicity." } |

At step 212 of the method of the present disclosure, the one or more hardware processors 104 generate, by using the one or more LLMs, a third summary (e.g., diagnosis summary and may be interchangeably used herein) based on the first summary and the second summary. The diagnosis summary for a particular user is extracted using the designed prompts that take temporal and non-temporal summary as an input for the same user. The prompts are provided below in Table 12 along with the example's diagnosis for Author1.

**Table 12**

| **Input** | | **System Prompt for Diagnosis Summary** | **User Prompt for Diagnosis Summary** | **Diagnosis Summary** |
|---|---|---|---|---|
| Non-Temporal Summary for Author1 | Temporal Summary for Author1 | You are an advanced language model trained to analyze and summarize text data related to mental state of a particular user. Given a data covering various features of a user based on the content posted by him/her on reddit, your task is to further summarize it concisely and provide the summary limited to 400 words covering every aspect with clear information such that when the summary is given to a mental health practitioner who works in accordance with DSM-5, it becomes helpful for him/her to recommend the therapy and behavior changes to the user based on it. Your task is specified to generate such a summary which covers all the necessary information regarding the mental health state of the user. | Summary: Dataframe.\n Review the provided data of the user's overall mental state covering several features. Summarize this further concisely and provide the summary in 400 words covering every aspect with clear information such that when the summary is given to a mental health practitioner who works in accordance with DSM-5, it becomes helpful for him/her to recommend the therapy and behavior changes to the user based on it. Your task is specified to generate such a summary which covers all the necessary information regarding the mental health state of the user. | Based on the provided data, smarmyslayer, a Reddit user, is experiencing a moderate to severe mental health condition. The primary causes of their distress are loneliness, social isolation, and academic struggles. Their language style is confessional and melancholic, indicating a high level of emotional vulnerability. The recurring themes in their posts revolve around feelings of loneliness, social isolation, and anxiety, which are characteristic of Major Depressive Disorder (MDD) as outlined in the DSM-5. The user's symptoms meet the criteria for MDD, with persistent feelings of sadness, hopelessness, and a lack of interest in activities they once enjoyed. Their mental health status is characterized by a pervasive sense of sadness, anxiety, and a sense of disconnection from others. The user's posts suggest a chronic and debilitating condition that has been ongoing for an extended period. The temporal patterns observed in the user's posts indicate a consistent pattern of distress over a four-month period, with no clear indication of improvement or worsening. The duration of their symptoms is concerning, as it suggests a prolonged period of suffering that may be impacting their daily life and relationships. A mental health practitioner working in accordance with the DSM-5 would likely diagnose smarmyslayer with Major Depressive Disorder and recommend evidence-based treatments such as cognitive-behavioral therapy and medication to alleviate their symptoms. |

At step 214 of the method of the present disclosure, the one or more hardware processors 104 generate, by using the one or more LLMs, one or more actionable health disorder specific recommendations based on an analysis of the third summary. The one or more actionable health disorder specific recommendations comprise at least one of a therapy, and one or more behavioral changes. For users dealing with daily stressors or issues stemming from lifestyle factors, the system 100 recommends practical behavioral changes. These include, for example, but are not limited to, adopting mindfulness techniques to enhance emotional regulation and stress management, engaging in regular physical exercise to improve overall mental and physical health, and implementing strategies to improve sleep patterns to ensure restorative rest. By providing these comprehensive recommendations, we aim to offer the user a balanced approach that combines professional support with actionable lifestyle modifications, thereby fostering long-term mental health and overall well-being. Table 13 depicts exemplary recommendations generated by the system 100 by using the third summary (e.g., the diagnosis summary):

**Table 13**

| **Input** | **System Prompt for Recommendations** | **User Prompt for Recommendations** | **Recommendations** |
|---|---|---|---|
| Based on the provided data, smarmyslayer, a Reddit user, is experiencing a moderate to severe mental health condition. The primary causes of their distress are loneliness, social isolation, and academic struggles. Their language style is confessional and melancholic, indicating a high | You are a Mental Health Practitioner working in accordance with DSM-5. | Summary : Dataframe.\n Review the provided summary of the user's behavior and mental health. Consider all the important information, and generate at the max three most suitable therapy recommendations and five actionable behavior modification suggestions separately that | Therapy Recommendations: |
| | | | 1. Cognitive Behavioral Therapy (CBT) |
| | Given the summary of a user's behavior and mental health trends, provide therapy recommendations and suggest behavior changes separately that could benefit the user. Consider all the relevant and | | |
| | | | 2. Interpersonal Therapy (IPT) |
| | | | 3. Psychodynamic Therapy |
| | | | Behavior Modification Suggestions: |
| | | | 1. Engage in regular physical activity to improve mood and reduce |
| level of emotional vulnerability. The recurring themes in their posts revolve around feelings of loneliness, social isolation, and anxiety, which are characteristic of Major Depressive Disorder (MDD) as outlined in the DSM-5. The user's symptoms meet the criteria for MDD, with persistent feelings of sadness, hopelessness, and a lack of interest in activities they once enjoyed. Their mental | important information mentioned in the summary and recommend at the max three most suitable therapies and five actionable behavior changes accordingly. | could help the user improve their well-being. Do not provide any extra explanations. | symptoms of depression. |
| | | | 2. Practice social skills and connect with others through online communities or in-person support groups. |
| | | | 3. Set realistic goals and prioritize self-care activities to increase sense of accomplishment and purpose. |
| | | | 4. Limit social media use and avoid comparing oneself to others to reduce feelings of inadequacy and isolation. |
| | | | 5. Keep a daily journal to process emotions and identify patterns and triggers of distress. |
| health status is characterized by a pervasive sense of sadness, anxiety, and a sense of disconnection from others. The user's posts suggest a chronic and debilitating condition that has been ongoing for an extended period. The temporal patterns observed in the user's posts indicate a consistent pattern of distress over a four-month period, with no clear indication of improvement or worsening. The duration of their symptoms is | | | |
| concerning, as it suggests a prolonged period of suffering that may be impacting their daily life and relationships. A mental health practitioner working in accordance with the DSM-5 would likely diagnose smarmyslayer with Major Depressive Disorder and recommend evidence-based treatments such as cognitive-behavioral therapy and medication to alleviate their symptoms. | | | |

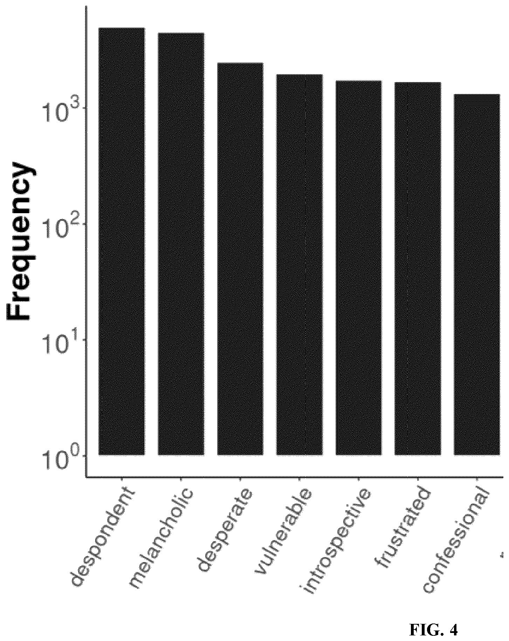

FIG. 4, with reference to FIGS. 1 through 3, depicts a graphical representation illustrating used language styles and tones by online users, in accordance with an embodiment of the present disclosure. The frequency of these language styles and tones provides valuable insights into the emotional landscape of users within the dataset. The prominence of negative tones like despondent and melancholic aligns with the high occurrence of depressive disorders and related mental health concerns, demonstrating a strong correlation between language use and the emotional or psychological state of users.

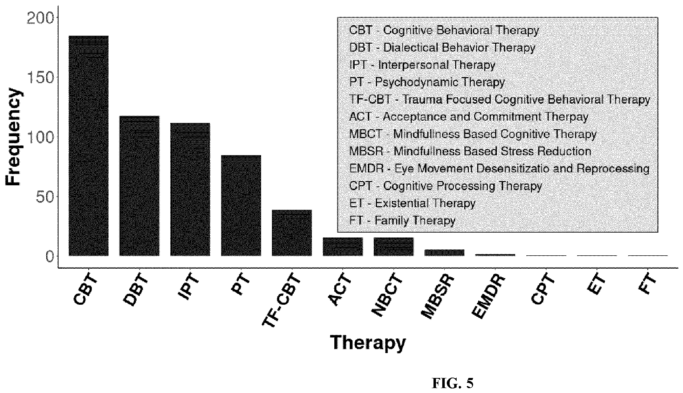

FIG. 5, with reference to FIGS. 1 through 4, depicts a graphical representation illustrating distribution of various therapies recommended to the users, in accordance with an embodiment of the present disclosure. It is observed that Cognitive Behavioral Therapy (CBT) emerged as the most common recommendation, highlighting its widespread applicability for various mental health issues. Following closely, Dialectical Behavior Therapy (DBT) was recommended, indicating its effectiveness in addressing emotional regulation and interpersonal effectiveness. Additionally, Interpersonal Therapy (IPT) was suggested, reflecting its relevance in improving social functioning and focusing on enhancing interpersonal relationships. Beyond these three, several other therapies were also recommended, demonstrating a diverse array of interventions tailored to meet the specific needs of users based on their diagnoses. This breadth of recommendations underscores the importance of personalized treatment approaches in mental health care.

In the present disclosure, embodiments provide a system and a method for the evaluation of multiple LLMs involving binary, multi-class, multi-label classification, and recommendation tasks from social media discussions related to mental health. Extracting domain-specific features from social media data is challenging due to its unstructured nature, making it difficult to identify relevant information accurately. Temporal information in text is often sparse, requiring a specialized approach for accurate capture. LLMs frequently overlook this, resulting in incomplete user profile summaries. By combining text creation time with temporal details extracted from the content, the system can build a chronological mental health history, which is essential for DSM-5 diagnosis. Collecting a user's text across various timelines and providing it to LLMs poses a challenge due to input token limitations, making it hard to include all relevant details. To address this, the system extracted key features from the text and used these features to create a comprehensive mental health summary for each user, ensuring important information is retained. The method of the present disclosure is not only robust in identifying a wide range of mental health disorders but also versatile enough to provide tailored recommendations for therapy and behavior changes. Although the system and method of the present disclosure present results for a sample dataset, the framework can be scaled up for much larger datasets, making it a valuable tool for large-scale mental health assessment and recommendations.

Moreover, careful prompt engineering of LLMs has proven effective in generating comprehensive insights, including disorder identification, severity assessment, recommendation of therapy and behavior changes. The experimental results were conducted as depicted in the graphs of FIGS. 4 and 5 through various LLMs being implemented by the system as known in the art. It is to be understood by a person having ordinary skill in the art or person skilled in the art various prompts may be provided to the LLMs depending upon their configurations and such prompts shall not be construed as limiting the scope of the present disclosure.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method, comprising:
receiving, via one or more hardware processors, an input data pertaining to a plurality of users (202);
filtering, by using one or more Large Language Models (LLMs) via the one or more hardware processors, the input data to obtain a filtered data specific to at least one health disorder pertaining to at least a subset of the plurality of users (204);
extracting, by using the one or more LLMs via the one or more hardware processors, a first set of features and a second set of features from the filtered data based on one or more context associated prompts (206);
creating, by using the one or more LLMs via the one or more hardware processors, one or more user profiles for the subset of the plurality of users based on the extracted first set of features and the extracted second set of features (208);
generating a first summary and a second summary based on the extracted first set of features and the extracted second set of features respectively for each user profile amongst the one or more user profiles (210);
generating, by using the one or more LLMs via the one or more hardware processors, a third summary based on the first summary and the second summary (212);
generating, by using the one or more LLMs via the one or more hardware processors, one or more actionable health disorder specific recommendations based on an analysis of the third summary (214).

2. The processor implemented method as claimed in claim 1, wherein the first set of features comprises at least one of (i) a duration, (ii) a frequency, (iii) a recurrence, and (iv) one or more explicit time and date mentions.

3. The processor implemented method as claimed in claim 1, wherein the second set of features comprises at least one of (i) a severity of the at least one health disorder, (ii) one or more factors impacting the at least one health disorder, and (iii) a language and tonality in the filtered data pertaining to the at least one health disorder.

4. The processor implemented method as claimed in claim 1, wherein the one or more actionable health disorder specific recommendations comprise at least one of a therapy, and one or more behavioral changes.

5. A system (100), comprising:
a memory (102) storing instructions;
one or more communication interfaces (106); and
one or more hardware processors (104) coupled to the memory (102) via the one or more communication interfaces (106), wherein the one or more hardware processors (104) are configured by the instructions to:
receive an input data pertaining to a plurality of users;
filter, by using one or more Large Language Models (LLMs), the input data to obtain a filtered data specific to at least one health disorder pertaining to at least a subset of the plurality of users;
extract, by using the one or more LLMs, a first set of features and a second set of features from the filtered data based on one or more context associated prompts;
create, by using the one or more LLMs, one or more user profiles for the subset of the plurality of users based on the extracted first set of features and the extracted second set of features;
generate, by using the one or more LLMs, a first summary and a second summary based on the extracted first set of features and the extracted second set of features respectively for each user profile amongst the one or more user profiles;
generate, by using the one or more LLMs, a third summary based on the first summary and the second summary;
generate, by using the one or more LLMs, one or more actionable health disorder specific recommendations based on an analysis of the third summary.

6. The system as claimed in claim 5, wherein the first set of features comprises at least one of (i) a duration, (ii) a frequency, (iii) a recurrence, and (iv) one or more explicit time and date mentions.

7. The system as claimed in claim 5, wherein the second set of features comprises at least one of (i) a severity of the at least one health disorder, (ii) one or more factors impacting the at least one health disorder, and (iii) a language and tonality in the filtered data pertaining to the at least one health disorder.

8. The system as claimed in claim 5, wherein the one or more actionable health disorder specific recommendations comprise at least one of a therapy, and one or more behavioral changes.

9. One or more non-transitory machine-readable information storage mediums comprising one or more instructions which when executed by one or more hardware processors cause:
receiving input data pertaining to a plurality of users;
filtering by using one or more Large Language Models (LLMs) the input data to obtain a filtered data specific to at least one health disorder pertaining to at least a subset of the plurality of users;
extracting by using the one or more LLMs, a first set of features and a second set of features from the filtered data based on one or more context associated prompts;
creating by using the one or more LLMs, one or more user profiles for the subset of the plurality of users based on the extracted first set of features and the extracted second set of features;
generating a first summary and a second summary based on the extracted first set of features and the extracted second set of features respectively for each user profile amongst the one or more user profiles;
generating by using the one or more LLMs, a third summary based on the first summary and the second summary;
generating by using the one or more LLMs, one or more actionable health disorder specific recommendations based on an analysis of the third summary.

10. The one or more non-transitory machine-readable information storage mediums as claimed in claim 9, wherein the first set of features comprises at least one of (i) a duration, (ii) a frequency, (iii) a recurrence, and (iv) one or more explicit time and date mentions.

11. The one or more non-transitory machine-readable information storage mediums as claimed in claim 9, wherein the second set of features comprises at least one of (i) a severity of the at least one health disorder, (ii) one or more factors impacting the at least one health disorder, and (iii) a language and tonality in the filtered data pertaining to the at least one health disorder.

12. The one or more non-transitory machine-readable information storage mediums as claimed in claim 9, wherein the one or more actionable health disorder specific recommendations comprise at least one of a therapy, and one or more behavioral changes.
